# EUROPEAN PATENT SPECIFICATION

(11) **EP 4 590 220 B1**
(45) Date of publication and mention of the grant of the patent: **10.06.2026**
(21) Application number: 23768792.6
(22) Date of filing: 05.09.2023
(51) Int. Cl.: A61B 18/14, A61B 18/18, A61B 18/00

(54) **ELECTROSURGICAL INSTRUMENT AND ELECTROSURGICAL APPARATUS**
ELEKTROCHIRURGISCHES INSTRUMENT UND ELEKTROCHIRURGISCHE VORRICHTUNG
INSTRUMENT ÉLECTROCHIRURGICAL ET APPAREIL ÉLECTROCHIRURGICAL

(30) Priority: 23.09.2022 GB 202213948
(43) Date of publication of application: 30.07.2025
(73) Proprietor: Creo Medical Limited, Chepstow, Wales NP16 5UH (GB)
(72) Inventor: ULLRICH, George Christian, Chepstow, NP16 5UH (GB); THOMAS, Steven, Chepstow, NP16 5UH (GB); JONES, Warren, Chepstow, NP16 5UH (GB); HANCOCK, Christopher Paul, Chepstow, NP16 5UH (GB); TURNER, Louis, Chepstow, NP16 5UH (GB); FITZSIMONS, Duncan James Foster, London Limited Pendragon House 65 London Road St. Albans Hertfordshire AL1 1LJ (GB)
(74) Representative: Mewburn Ellis LLP
(86) International application number: PCT/EP2023/074243
(87) International publication number: WO 2024/061611

(56) References cited:
- US-A1- 2016 296 273
- US-A1- 2016 331 455
- US-A1- 2020 253 664

## Description

### FIELD OF THE INVENTION

The invention relates to an electrosurgical instrument for sealing and cutting tissue. The electrosurgical instrument is configured to grasp biological tissue and deliver microwave energy into the grasped tissue to seal the tissue by coagulation or cauterisation. The electrosurgical instrument may be used to apply pressure to close one or more blood vessels before applying electromagnetic radiation (preferably microwave energy) to seal the blood vessel(s). The electrosurgical instrument may also be arranged to cut, e.g. separate or divide, the vessel or surrounding tissue after coagulation or sealing, e.g. using radiofrequency (RF) energy and/or a mechanical cutting element, such as a blade. The invention may be applied to a vessel sealer for use in laparoscopic surgery or open surgery as well as to an endoscopic instrument.

The invention also relates to an electrosurgical apparatus for sealing and cutting tissue which comprises a generator unit for generating radiofrequency and/or microwave electromagnetic energy, and the electrosurgical instrument.

### BACKGROUND TO THE INVENTION

Electrosurgical instruments for delivering heat energy into grasped biological tissue are known. For example, it is known to deliver microwave energy from a bipolar electrode arrangement in the jaws of a forceps. The microwave energy may be used to seal a vessel by thermal denaturation of extracellular matrix proteins (e.g. collagen) within the vessel wall. The heat energy may also cauterise the grasped tissue and facilitate coagulation.

Such devices typically find application on the end of minimally invasive surgical laparoscopic tools but can equally find use in other clinical procedural areas such as gynaecology, endourology, gastrointestinal surgery, ENT procedures, or endoscopic procedures. Depending on the context of use, these devices can have differing physical construction, size, scale and complexity.

For example, a gastrointestinal instrument might be nominally of 3 mm diameter mounted on to the end of a very long flexible shaft. In contrast, a laparoscopic instrument may be used on the end of an industry standard nominal 5mm or 10mm diameter rigid or steerable steel shaft.

US 6,585,735 describes an endoscopic bipolar forceps in which the jaws of the forceps are arranged to conduct bipolar energy through the tissue held therebetween.

EP 2 233 098 describes microwave forceps for sealing tissue in which the sealing surfaces of the jaws include one or more microwave antennas for radiating microwave energy into tissue grasped between the jaws of the forceps.

WO 2015/097472 describes electrosurgical forceps in which one or more pairs of non-resonant unbalanced lossy transmission line structures are arranged on the inner surface of a pair of jaws.

US 2020/253664 A1 refers to an electrosurgical apparatus comprising an electrosurgical forceps instrument that combines a robust jaw opening mechanism with a microwave energy delivery mechanism. The instrument includes a rigid bracket mounted at a distal end of a flexible shaft, wherein a pair of jaws are pivotably mounted on the rigid bracket.

US 2016/331455 A1 discloses electrosurgical forceps in which one or more pairs of non-resonant unbalanced lossy transmission line structures are arranged on the inner surfaces of the jaws of the forceps provide both (i) active and return electrodes for a radiofrequency (RF) signal, and (ii) lossy structures for delivering a microwave signal into biological tissue in conjunction with a mechanical gripping arrangement for applying pressure to material held within the jaws.

### SUMMARY OF THE INVENTION

At its most general, the present invention provides an electrosurgical instrument that can seal biological tissue, such as (blood) vessels, using a confined microwave field that can yield a well-defined seal location with low thermal margin. Moreover, the electrosurgical instruments may provide additional functionality, such as cutting using the emission of radiofrequency electromagnetic energy to enable fine tissue cutting and dissection to be performed. With these additional functions, fewer device interchanges may be needed during a procedure.

The electrosurgical instruments disclosed herein may be used in any type of surgical procedure, but it is expected to find particular utility for non-invasive or minimally invasive procedures. For example, the device may be configured to be introduced to a treatment site through an instrument channel of a surgical scoping device, such as a laparoscope or an endoscope.

In one example, the electrosurgical instrument may include features of an electrosurgical resector tool because it comprises a pair of blade elements that provide a scissor-like mechanism that can provide two complimentary modalities: (i) a scissor-type cut performed on tissue grasped between the blade elements and/or jaws using a combination of RF energy and applied pressure, and (ii) a coagulation or vessel sealing operation performed on tissue grasped between the blade elements and/or the jaws using a combination of microwave energy and applied pressure. Moreover, the RF and/or microwave energy may be supplied in any of these modalities at a power level sufficient to cause tissue ablation. By suitable configuration of a pair of electrodes on the blade elements, the supplied RF or microwave energy in each of these operational modalities can be focussed in the region required. In an embodiment, only one blade element includes two electrodes, such that a total of two electrodes are present. In another embodiment, each blade element includes two electrodes, such that a total of four electrodes are present.

According to a first aspect of the present invention, there is provided an electrosurgical instrument for sealing and cutting tissue which comprises an instrument shaft, a first jaw, a second jaw, a first blade element, and a second blade element. The instrument shaft comprises a (coaxial) transmission line for conveying microwave and radiofrequency electromagnetic energy. The first jaw is attached to the instrument shaft and includes a first surface. The second jaw is attached to the instrument shaft and includes a second surface. The first jaw and the second jaw can be moved between an open position, in which the tissue can be inserted between the first surface and the second surface, and a closed position, in which the first and second surfaces are brought together to clamp and/or grasp tissue therebetween. The first jaw includes a first recessed portion in which the first blade element can be received in a rest position. The second jaw includes a second recessed portion in which the second blade element can be received in the rest position. The first blade element and/or the second blade element are configured to be moved towards each other so that they contact each other defining a cutting position. The first blade element and/or the second blade element comprises a longitudinally extending planar dielectric body having a first electrode on a first laterally facing surface thereof, and a second electrode spaced away from the first electrode and electrically isolated therefrom by at least the planar dielectric body. In the cutting position, the second blade element (e.g. first laterally facing surface of the second blade element) lies adjacent to the first laterally facing surface of the first blade element, for example, in the cutting position, the first blade element and second blade element lie alongside each other in a lateral direction that is normal to the rotation plane. The first electrode and the second electrode are operable: (1) as active and return electrodes for delivering radiofrequency electromagnetic energy and (2) as microwave electrodes for emitting microwave electromagnetic energy.

In an embodiment, in the closed position of the first jaw and the second jaw and the rest position of the first blade element and the second blade element, the first blade element and the second blade element are spaced from each other. In another embodiment, in the closed position of the first jaw and the second jaw and the rest position of the first blade element and the second blade element, the first blade element and the second blade element touch. For example, the first electrode of the first blade element may contact the first electrode of the second blade element.

As the first blade element and the second blade element lie adjacent to or alongside each other in the cutting position and/or the rest position, the first blade element and the second blade element may not have the ideal configuration to clamp or grasp tissue. To this end, the first jaw and the second jaw are provided whose first surface and second surface, respectively, may be used to clamp and grasp tissue in the rest position of the first blade element and the second blade element. Further, the first jaw and the second jaw may be used to fixate (e.g. hold or secure) the tissue during cutting of the tissue by the first blade element and the second blade element.

In the closed position of the jaws and the rest position of the blade elements, the first jaw and the second jaw may (completely) surround the first blade element and the second blade element so that the first jaw and the second jaw (which may include electrically conductive material) may shield the first blade element and the second blade element so that little or no electromagnetic radiation emitted by the first blade element and/or the second blade element is emitted by the electrosurgical instrument.

The first blade element and the second blade element may contact each other in the cutting position and when the first blade element and the second blade element are moved away from the cutting position. For example, proximal end portions of the first blade element and the second blade element may contact each other since these proximal end portions are close to a pivot axis so that, even in an open position of the blade elements, the blade elements are not spaced apart at these proximal end portions. However, at a distal end portion, the blade elements are spaced away from each other. This is due to the scissors-configuration of the blade elements.

In use, the electrosurgical instrument may thus perform vessel/tissue sealing and vessel/tissue dividing. Vessel/tissue sealing is typically the application of pressure to squash the walls of a biological vessel together, followed by the application of some form of thermal energy. In the invention, the thermal energy is applied by the first and second electrodes to the gripped tissue using the microwave electromagnetic energy. The pressure to the tissue can be applied by the first and/or second electrodes (e.g. the first and second sealing areas) and/or other parts of the first and second jaws. The applied electromagnetic energy disrupts/denatures the tissue cells and forms an amalgam of collagen predominant in vessel/tissue walls, which effectively bonds the vessel/tissue walls together. With time, post operatively, cellular recovery and regrowth occurs to reinforce the seal further. Vessel/tissue dividing is a process of cutting through a continuous biological vessel/tissue to separate it into two pieces. It is normally performed after a vessel/tissue is first sealed. Vessel/tissue dividing is performed by the cutting device, which is discussed in more detail below. As described above, the vessel/tissue dividing is locally offset from the vessel/tissue sealing. In particular, the vessel/tissue dividing occurs between the vessel/tissue seals. Stated differently, the instrument is configured such that vessel/tissue dividing occurs at a different location to vessel/tissue sealing.

Herein, the terms "proximal" and "distal" refer to the ends of the electrosurgical instrument, the shaft, and/or the coaxial transmission line further from and closer to a treatment site respectively. Thus, in use the proximal end is closer to a generator unit for providing the RF and/or microwave energy, whereas the distal end is closer to the treatment site, i.e. the patient.

The term "conductive" is used herein to mean electrically conductive, unless the context dictates otherwise.

The term "longitudinal" used below refers to the direction along the instrument channel parallel to the axis of the coaxial transmission line. The term "lateral" refers to a direction that is perpendicular to the longitudinal direction. The term "inner" means radially closer to the centre (e.g. axis) of the instrument channel. The term "outer" means radially further from the centre (axis) of the instrument channel.

The term "electrosurgical" is used in relation an instrument, apparatus or tool which is used during surgery and which utilises radiofrequency (RF) electromagnetic (EM) energy and/or microwave EM energy. Herein, RF EM energy may mean a stable fixed frequency in a range 10 kHz to 300 MHz, preferably in a range from 100 kHz to 5MHz, and more preferably in a range from 360 to 440 kHz. The microwave EM energy may mean electromagnetic energy having a stable fixed frequency in the range 300 MHz to 100 GHz. The RF EM energy should have a frequency high enough to prevent the energy from causing nerve stimulation. In use, the magnitude of the RF EM energy and the duration for which it is applied may be selected to prevent the energy from causing tissue blanching or unnecessary thermal margin or damage to the tissue structure. Preferred spot frequencies for the RF EM energy include any one or more of: 100 kHz, 250 kHz, 400 kHz, 500 kHz, 1 MHz, 5 MHz. Preferred spot frequencies for the microwave EM energy include 915 MHz, 2.45 GHz, 5.8 GHz, 14.5 GHz, 24 GHz. 2.45 GHz and/or 5.8 GHz may be preferred.

The microwave electromagnetic energy and the radiofrequency electromagnetic energy may be conveyed along a common signal pathway through the instrument shaft. For example, a coaxial cable may provide the common signal pathway for conveying both the microwave energy and the radiofrequency energy. In this arrangement, the transmission line may comprise an inductive filter for blocking the microwave energy from the cutting element, and a capacitive filter for blocking the radiofrequency energy from the first and second electrodes. In an alternative arrangement, the radiofrequency energy and microwave energy are conveyed along separate pathways within the instrument shaft (the transmission line includes separate pathways), wherein the inductive filter and capacitive filter are provided at a proximal end of the instrument shaft, e.g. in a handle. For example, a coaxial cable is provided for conveying the microwave electromagnetic energy while two or more wires are provided for conveying the radiofrequency electromagnetic energy.

The instrument shaft may be dimensioned to fit within an instrument channel of a surgical scoping device. The surgical scoping device may be a laparoscope or an endoscope. Surgical scoping devices are typically provided with an insertion tube that is a rigid or flexible (e.g. steerable) conduit that is introduced into a patient's body during an invasive procedure. The insertion tube may include the instrument channel and an optical channel (e.g. for transmitting light to illuminate and/or capture images of a treatment site at the distal end of the insertion tube). The instrument channel may have a diameter suitable for receiving invasive surgical tools. The diameter of the instrument channel may be equal to or less than 13 mm, preferably equal to or less than 10 mm, and more preferably, especially for flexible insertion tubes, equal to or less than 5 mm.

The instrument shaft and the transmission line may be flexible so that they can be inserted into the instrument channel of the scoping device. Further, the transmission line may be arranged within a lumen of the shaft. The instrument shaft may cover and/or shield the transmission line. The transmission line may extend from a distal end to a proximal end of the electrosurgical instrument. In particular, the transmission line electrically connects the first electrode and the second electrode to the generator unit.

The electrosurgical instrument discussed herein may find applicability in other tissue welding techniques. For example, the energy delivery structure may be used as an alternative to staples. In some abdominal procedures, staple guns are used to deliver 50 to 100 small staples that are fired simultaneously between jaws that can have a length of 70 mm or more, or from an annular jawed arrangements with diameters of 20 to 50 mm. In this type of application multiple antenna structures such as those discussed herein may be used to cover the required length. The antenna structures may be arranged in any number of array forms to be activated simultaneously, sequentially or progressively in a suitable manner.

The first jaw and/or the second jaw may be movable relative to their instrument shaft. The first jaw and/or the second jaw may be attached to the instrument shaft via a joint or hinge. The joint may include a pivot axis around which the first jaw and/or the second jaw may rotate. The first jaw and/or the second jaw may be activated by one or more actuation rods or control wires respectively connected to the first jaw and/or the second jaw. The one or more actuation rods or control wires may extend within the instrument shaft to a proximal end of the electrosurgical instrument. The one or more actuation rods may be connected to a handle with which the first and/or second jaws can be actuated, e.g. opened and/or closed. The electrosurgical instrument comprises an actuation mechanism which converts a back-and-forth movement of the actuation rod(s) or control wire(s) in a rotational movement of the first jaw and/or the second jaw.

For example, both jaws can be movable, e.g. rotatable around a (common) pivot axle. In another embodiment, one of the jaws is fixed to the shaft and the other jaw is movable relative to the one jaw.

In the open position, the first jaw and the second jaw are (maximally) spaced apart so that there is a free space between the first surface of the first jaw and the second surface of the second jaw. In this way, tissue can be inserted between the first surface and the second surface in the open position. Usually, the first jaw and the second jaw are moved towards the tissue such that the tissue is pushed into the space between the first surface and the second surface in the open position of the first jaw and the second jaw.

By moving the first jaw and/or the second jaw from the open position to the closed position, the tissue between the first surface and the second surface can be grasped and/or clamped between the first surface and the second surface. In this way, the tissue can be fixed between the first surface and the second surface in the closed position. The first surface and the second surface are the faces of the first jaw and the second jaw, respectively, that face each other in the open and/or closed position.

The pair of jaws may be pivotable relative to each other about a hinge axis that lies transverse to a longitudinal axis of the coaxial transmission line. In one example, the pair of jaws comprises a static jaw that is fixed relative to the instrument shaft, and a movable jaw that is pivotably mounted relative to the static jaw to open and close the gap between the opposing inner surfaces. The energy delivery structure may be disposed on the inner surface of the static jaw. In another example, both jaws are arranged to pivot with respect to the instrument shaft, e.g. in a symmetrical forceps-type or scissors-type arrangement. Relative movement of the pair of jaws may be controlled from a handle at a proximal end of the instrument shaft. A control rod or control wires may pass through the instrument shaft to operably couple an actuation mechanism on the handle to the pair of jaws.

In another example, the pair of jaws may be arranged to move relative to one another in a manner that maintains the inner surfaces thereof in an aligned, e.g. parallel, orientation. This configuration may be desirable for maintaining a uniform pressure on grasped tissue along the length of the jaws. One example of such a closure mechanism is disclosed in WO 2015/097472.

The first jaw and/or the second jaw may have a Maryland configuration. This can include that the first jaw and the second jaw are not straight but bent/curved, e.g. forming an arc or an S-shape in a side view.

The first jaw and/or the second jaw may include electrically conductive material that has a shape of half-shells. In the closed position, the half-shells of the first jaw and the second jaw may substantially or completely surround the first blade element and the second blade element. Further, the first jaw and the second jaw, optionally the respective half-shells, may be configured to clamp or grasp tissue in the closed position. So, the first surface and/or the second surface are configured and/or shaped to provide a surface area of sufficient size and/or surface texture to clamp, hold, and/or grasp tissue.

The half-shells of the first jaw and/or the second jaw may define the first recessed portion and the second recessed portion, respectively. In a closed position, the first blade element and the second blade element can be received in the first jaw and the second jaw, respectively. In the closed position, a gap may be provided between the first surface (a surface of the first jaw that faces the second jaw and is configured to clamp and grasp tissue) and the second surface (a surface of the second jaw that faces the first jaw and is configured to clamp and grasp tissue). The gap may be filled with the tissue. The gap may have a dimension configured to clamp thin pieces of tissue. In the rest position, the first blade element and/or the second blade element are in contact with the first jaw and/or the second jaw, respectively.

In an alternative embodiment, the first surface and the second surface are in contact with each other in the closed position (and in the absence of tissue). In this case, the first blade element and the second blade element may contact each other when the first blade element and the second blade element are in the rest position.

The first recessed portion and/or the second recessed portion may be delimited by an electrically conductive material (e.g. the half-shell). For example, the conductive material of the first jaw is point-symmetric to the conductive material of the second jaw in the closed position. The arrangement of the first blade element may be point-symmetric to the second blade element in the closed position and when the first and second blade elements are arranged in the first and second recessed portion, respectively.

In one example, the pair of blade elements (i.e. the first and the second blade elements) provide a scissor-like mechanism that can provide two complimentary modalities: (i) a scissor-type cut performed on tissue grasped between the blade elements using a combination of radiofrequency energy and applied pressure, and (ii) a coagulation or vessel sealing operation performed on tissue grasped by the first jaw and the second jaw between the blade elements using a combination of microwave energy and applied pressure. As the clamping and/or grasping of the tissue is predominantly effected by the first and second jaws (and not by the pair of blade elements or to a much lesser degree), the tissue can be more effectively clamped before and/or during cutting using the blade elements.

Moreover, the radiofrequency and/or microwave energy may be supplied in any of these modalities at a power level sufficient to cause tissue ablation. By suitable configuration of one or two pairs of electrodes on the blade elements, the supplied radiofrequency and/or microwave energy in each of these operational modalities can be focussed on the region required.

The pair of electrodes (i.e. the first electrode and the second electrode) may both be on the same blade element. Further, each blade element may include a first electrode and a second electrode.

In this structure, the first and second blade elements may resemble a scissors-type closure mechanism. Thus, the second blade element may be arranged to slide past the first blade element during movement between the rest position (in which the blade elements are arranged in the respective recessed portions) and the cutting position, e.g. to effect mechanical cutting through application of a shearing force. In the cutting position, the blade elements contact each other and/or at least partially overlap each other. For example, the first electrode of the first blade element contacts and/or at least partially overlaps with the first electrode of the second blade element.

The first blade element and/or the second blade element may be movable relative to each other (and/or relative to the first jaw and/or the second jaw, respectively) in a plane parallel to a plane defined by the planar dielectric body, e.g. plane defined by the first laterally facing surface the planar dielectric body. The first electrode and/or second electrode may be disposed at a cutting interface.

The planar dielectric body may be a separate piece of material mounted on, e.g. adhered or otherwise affixed to, a support arm. The planar dielectric body may be formed from ceramic (e.g. alumina). Herein, reference to "planar" material may mean a flat piece of material having a thickness that is substantially less that its width and length. The planar dielectric body may have a length dimension aligned in the longitudinal direction, a thickness dimension aligned in a lateral direction, and a width dimension orthogonal to both the length and thickness dimensions. A plane of the planar dielectric body is that in which the length and width dimensions lie, i.e. a plane orthogonal to the thickness dimension.

The first electrode may be a conductive material (e.g. gold) deposited or otherwise mounted on the first laterally-facing surface of the planar dielectric body. The first laterally-facing surface of the planar dielectric body may be exposed at the cutting interface. Therefore, the first electrode may be exposed on the cutting edge. The second laterally-facing surface of the planar dielectric body that faces in an opposite direction to the first laterally-facing surface may include the second electrode. The second electrode may be a conductive material (e.g. gold) deposited or otherwise mounted on the second laterally-facing surface of the planar dielectric body. This may assist in providing uniform energy delivery at the cutting interface. The other blade element may include the first electrode and/or the second electrode as described in connection with the first blade element.

The first electrode of the first blade element and the first electrode on the second blade element may contact each other or lie adjacent opposing each other in the cutting position. This means that the first laterally-facing surface of the planar dielectric body of the first blade element faces the first laterally-facing surface of the planar dielectric body of the second blade element in the cutting position.

In this arrangement, radiofrequency electromagnetic energy applied to the electrodes flows preferentially between the first and second laterally-facing surfaces of the planar dielectric body. Similarly, if microwave energy is applied while the blade elements are open, a microwave field emitted by the electrodes has a high field strength across the planar dielectric body.

Stated differently, the second electrode is separated from the first electrode along much of its length by the planar dielectric body. If radiofrequency electromagnetic energy is applied, the radiofrequency electromagnetic energy preferentially flows around a side edge of the blade elements, which facilitates a radiofrequency-only cut.

The first electrode of the first blade element may be electrically connected to the first electrode of the second blade element either directly or via an electric connection to the same conductor of the transmission line. The second electrode of the first blade element and the first electrode of the second blade element either have a direct electrical connection to each other or each have an electric connection to the same conductor of the transmission line. Further, the second electrodes of the first and second blade elements may be on the same electrical potential as the conductive material of the first and second jaws. In other words, the second electrodes of the first and second blade elements may not be electrically isolated from the first and second jaws. However, the first electrodes of the first and second blade elements are electrically isolated from the first jaw and the second jaw.

In an optional embodiment, the electrosurgical instrument further comprises a joint having a pivot axis. Optionally, the first jaw, the second jaw, the first blade element and/or the second blade element are rotatable around the pivot axis.

The first blade element and the second blade element may be rotatably mounted to the joint. For example, the joint may provide a clevis-type structure that supports a pivot axle defining the pivot axis. The first jaw, the second jaw, the first blade element and the second blade element may all be rotatably attached to the pivot axle. This means that the first jaw and the first blade element can be aligned with each other in any rotational position and the second jaw and the second blade element can be aligned with each other in any rotational position. For example, the first blade element can be maintained in the rest position (i.e. received in the first recessed portion) while moving the first jaw and the first blade element. Similarly, the second blade element can be maintained in the rest position (i.e. received in the second recessed portion) while moving the second jaw and the second blade element.

Further, the first blade element can be moved independently from the first jaw. So, a movement of the first jaw may not result in a movement of the first blade element and vice versa. The second blade element can be moved independently from the second jaw. So, a movement of the second jaw may not result in a movement of the second blade element and vice versa.

The electrical connection between the transmission line and the second electrode may pass through the joint. For example, the pivot axle may be formed from a conductive material, and the insulator coating of the fist and/or second blade element may be removed where they respectively contact the pivot axle.

Alternatively, the first blade element and/or the second blade element may be mounted to the first jaw and/or the second jaw, respectively, via a pivot connection.

In general, the movement between the first blade element and/or the second blade may be rotational or translational or a combination of the two.

In another example, it may be beneficial for a gap between the electrodes to be uniform once tissue is grasped therebetween, e.g. to ensure that the energy supplied is uniform along the length of the blade elements. In this example, the pair of blade elements may occupy a position in the cutting position in which the second blade element lies parallel to the first blade element but spaced therefrom to define a gap therebetween.

The actuator may comprise a control rod slidably mounted in the instrument shaft. The control rod may have an attachment feature engaged with the first blade element and/or the second blade element, whereby a longitudinal movement of the control rod in the shaft causes movement of the first blade element and/or the second blade element in the shaft, the first jaw, and/or the second jaw. The attachment feature may be a hook or any suitable engagement for transmitting push and pull forces into a rotational movement of the first blade element and/or the second blade element.

In one example, the first blade element and/or the second blade comprise a cam surface against which the respective control rod acts to drive movement of the first blade element and/or the second blade relative to each other. The cam surface may be engageable only during a final stage of the closure operation, e.g. to provide an additional force boost to complete the closure. In one example, the cam surface may be provided by a slot in the movable portion. The attachment feature comprises an engagement portion for being locating in the slot. A cam action may be provided by the engagement portion sliding along the slot.

In an optional embodiment, the first blade element is movable relative to the first jaw and/or the second blade element is movable relative to the second jaw.

This configuration means that the first blade element can be positioned protruding from the first jaw and the second blade element can be positioned protruding from the second jaw. So, it is possible to further advance the first blade element and the second blade element from the rest position when the first jaw and the second jaw are in the closed position. In other words, it is possible to cut tissue (move the first blade element and the second blade elements towards the cutting position) in the closed position of the first jaw and the second jaw. As described above, this is helpful for cutting tissue while clamping the tissue in the closed positions of the first jaw and the second jaw.

In an optional embodiment, in the rest position, the first blade element is flush with a section of the first surface or the entire first surface and/or the second blade element is flush with a section of the second surface or the entire second surface.

In the rest position, the first blade element and/or the second blade element may be flush with sections of the first surface and the second surface, respectively. For example, the first blade element and/or the second blade element may be flush with the section of the respective jaws that is directly adjacent to the respective first blade element and/or a respective second blade element.

In an optional embodiment, the first jaw is point-symmetrical to the second jaw.

This means that the first jaw may have the same configuration, shape, and/or design as the second jaw. However, the first and second jaws are positioned in different orientations. This may simplify the production of the first and second jaws.

In an optional embodiment, the first jaw and/or the second jaw includes a shell and a filler portion. Optionally, the filler portion is arranged in a cavity defined by the shell. Further optionally, the shell and the filler portion define the first recessed portion and/or the second recessed portion, respectively.

The shell and the filler portion may form the above-described half-shells. The shell may be made from an electrically conductive material, such as metal or a metal alloy. The filler portion may be made from a dielectric material. The first jaw and/or the second jaw may solely consist of the shell and the filler portion. The filler portion may have a volume then is equivalent to the volume of the blade element. The shell may define a cavity within which two blade elements can be virtually arranged. One of these two virtually arrangeable blade elements is replaced by the filler portion. Thus, a side surface of the filler portion may be adjacent and/or facing the first laterally facing surface of the respective blade element. The other surfaces of the blade element may face inner surfaces of the shell. The filler portion and the shell may be a unitary or single component.

In an optional embodiment, in the rest position, the first electrode is completely exposed and configured to contact tissue, e.g. in the closed position.

In a further optional embodiment, in the rest position, a portion of the planar dielectric body protrudes from (e.g. past or beyond) the filler portion and the first electrode is arranged on the protruding portion of the planar dielectric body.

In the rest position, a portion of the first laterally facing surface of the first blade element and/or the second blade element may be exposed because this is not covered by the filler portion in the rest position. The first electrode of the first blade element and/or the second blade element may be (completely) arranged on this exposed portion of the first laterally facing surface.

In an optional embodiment, the second electrode is located on the second laterally facing surface of the planar dielectric body of the first blade element and/or the second blade element.

So, the first electrode and the second electrode of the first blade element are spaced apart by a section of the planar dielectric body that is exposed along the first surface and/or faces the second surface. Similarly, the first electrode and the second electrode of the second blade element are spaced apart by a section of the planar dielectric body that is exposed along the second surface and/or faces the first surface. In other words, a thickness of the planar dielectric body defines the distance between the first electrode and the second electrode.

In an optional embodiment, the first blade element is point-symmetrical to the second blade element in the rest position and the closed position.

This means that the first blade element may have the same configuration, shape, and/or design as the second blade element. However, the first and second blade elements are positioned in different orientations. This may simplify the production of the first and second blade elements. For example, in the closed position, the first blade element is positioned opposite of the filler portion of the second jaw and the second blade element is positioned opposite of the filler portion of the first jaw.

In an optional embodiment, the first blade element and/or the second blade element each include a support arm to which the planar dielectric body is attached. Optionally, the support arm forms a part of an electrical connection between the transmission line and the second electrode.

The support arm may be made from an electrically conductive material, such as metal or a metal alloy. The support arm may have an L-shape in a cross-sectional view of the respective blade element. The support arm may cover or be in direct contact with two surfaces of the planar dielectric body, e.g. the second laterally facing surface. The surfaces of the planar dielectric body not covered or contacted by the arm support may include the first laterally facing surface and the surface facing the respective other jaw or blade element.

As the support arm may contact the second laterally facing surface on which the second electrode is positioned, the second electrode can be in contact with the support arm. As the support arm can be electrically conductive, the support arm and the second electrode can be on the same electrical potential. In fact, the electrical connection of the second electrode to the transmission line may be made via the support arm.

In an optional embodiment, the support arm is attached to the joint rotatably around the pivot axis.

The first blade element and/or the second blade element may be (solely) connected to the pivot axle via the respective support arm.

In an optional embodiment, the second electrode is arranged between the planar dielectric body and the support arm and is exposed between the planar dielectric body and the support arm to that the second electrode is configured to contact tissue.

The second electrode can be sandwiched between the planar dielectric body and the support arm wherein a side surface of the second electrode is exposed in a gap between the support arm and the planar dielectric body. The first electrode and/or the second electrode may be a strip of a highly conductive material, such as gold. Because of this, most of the electromagnetic energy that is emitted by the first electrode and the second electrode is emitted by these strips and not from other electrically conductive components that are electrically connected to one of the electrodes, e.g. the second electrode.

According to a second aspect of the present invention, there is provided an electrosurgical apparatus for sealing and cutting tissue which comprises a generator unit for generating radiofrequency and/or microwave electromagnetic energy and the electromagnetic electrosurgical instrument as described above. The transmission line is configured to convey the radiofrequency and/or microwave electromagnetic energy from the generator unit to first electrode, the second electrode and/or the third electrode.

The generator unit may be configured to generate electromagnetic energy of a fixed single frequency or of a plurality of fixed single frequencies. Alternatively or additionally, the generator unit may be tuneable to generate electromagnetic energy of various frequencies, for example in a continuous range of frequencies between a minimum frequency and a maximum frequency. The generator unit may be connected to a power supply which provides the energy for generating the radiofrequency electromagnetic energy and/or microwave electromagnetic energy.

The generator unit is electrically and/or electronically (directly or indirectly) connected to the transmission line. Optionally, the generator unit generates the radiofrequency energy and/or microwave energy which is conveyed by the transmission line to the first and second electrodes where the radiofrequency energy and/or microwave energy is radiated into the treatment zone.

In an optional embodiment, the generator unit is configured to simultaneously generate microwave electromagnetic energy of the first frequency and microwave electromagnetic energy of a second frequency.

For example, the generator unit includes a generator that is configured to simultaneously generate electromagnetic energy of two different (fixed) frequencies.

Alternatively, the generator unit includes a first generator for generating electromagnetic energy of the first frequency and a second generator for generating electromagnetic energy of the second frequency. The output of the first generator and output of the second generator can be combined using a multiplexer.

The multiplexer may be a diplexer and can combine the input from various sources into one output. For example, a multiplexer (or diplexer) is used to combine the output of first and second generators to a single output which is connected or coupled to the transmission line.

In an optional embodiment, the generator unit is configured to simultaneously or alternatingly generate microwave electromagnetic energy of the first frequency and radiofrequency electromagnetic energy of a third frequency.

The generator unit may include a first generator for generating electromagnetic energy of the first frequency, a second generator for generating electromagnetic energy of the second frequency, and/or a third generator for generating electromagnetic energy of the third frequency. The output of the first generator and the output of the second generator may be combined as described above using a multiplexer.

The output of the third generator may be combined with the output of the multiplexer using a combiner which can include a switch for alternatingly switching between outputting the output of the multiplexer and outputting the output of the third generator. The combiner may include an additional multiplexer for combining the output of the multiplexer and the output of the third generator to simultaneously emit electromagnetic energy of the first frequency, the second frequency, and the third frequency. In this case, microwave sealing and radiofrequency cutting can be simultaneously effected.

If switching between the output of microwave energy and radiofrequency energy is possible, this can be used for sealing the tissue using microwave energy and then subsequently cutting the tissue using radiofrequency energy. Alternatively, the switch between the output of microwave energy and radiofrequency energy can be executed repeatedly and rapidly providing near simultaneous cutting and sealing.

### BRIEF DESCRIPTION OF THE DRAWINGS

Embodiments of the invention are described in detail below with reference to the accompanying drawings, in which:
Fig. 1 shows a schematic view of an embodiment of an electrosurgical apparatus;
Figs. 2a and 2b show perspective views of an embodiment of an electrosurgical instrument of the electrosurgical apparatus shown in Fig. 1, wherein the electrosurgical instrument is in an open position of the jaws and in a rest position of the blade elements in Fig. 2a and the electrosurgical instrument is in the open position of the jaws and between the rest position and a cutting position of the blade elements in Fig. 2b; and
Fig. 3 shows a cross-sectional view of a simulated emission distribution emitted by the electrosurgical instrument of Figs. 2a and 2b in a closed position of the jaws and the rest position of the blade elements.

### DETAILED DESCRIPTION; FURTHER OPTIONS AND PREFERENCES

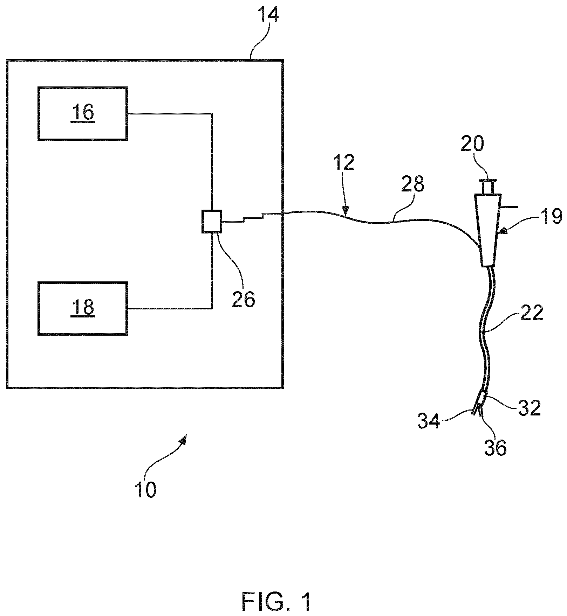

The present invention relates to an electrosurgical instrument and apparatus capable of delivering microwave energy to seal tissue (e.g. blood vessels) and/or of cutting the tissue. The electrosurgical instrument and apparatus may be used in open surgery but may find particular use in procedures where there is restricted access to the treatment site. For example, the electrosurgical instrument of the invention may be adapted to fit within the instrument channel of a surgical scoping device i.e. laparoscope, endoscope, or the like. Fig. 1 shows a schematic view of an electrosurgical apparatus 10 in which the electrosurgical instrument of the invention may be used.

Fig. 1 is a schematic diagram of an electrosurgical apparatus 10 that is an embodiment of the invention. The electrosurgical apparatus 10 is arranged to treat biological tissue using radiofrequency (RF) and/or microwave electromagnetic (EM) energy delivered from an electrosurgical instrument 12. The electromagnetic energy emitted by the electrosurgical instrument 12 into a treatment zone can be used to coagulate, cut, and/or ablate tissue in the treatment zone.

The electrosurgical apparatus 10 further comprises a generator unit 14 which can controllably supply radiofrequency and/or microwave electromagnetic energy to the electrosurgical instrument 12. The generator unit 14 may include a first generator 16 and a second generator 18. Suitable generators for this purpose are described in WO 2012/076844. The generator unit 14 may be arranged to monitor reflected signals received back from the electrosurgical instrument 12 in order to determine an appropriate power level for delivery. For example, the generator unit 14 may be arranged to calculate an impedance seen at the electrosurgical instrument 12 in order to determine an optimal delivery power level.

The electrosurgical apparatus 10 further comprises a surgical scoping device 19, such as a bronchoscope, endoscope, gastroscope, laparoscope or the like. The scoping device 19 may include a handpiece 20 and a flexible shaft 22. The handpiece 20 may include means for guiding the flexible shaft 22 through a cavity of a body. For example, the handpiece 20 can include means for moving a distal end of the flexible shaft 22 to change direction of the distal end of the flexible shaft 22. This helps manoeuvring the flexible shaft 22 through the cavity of the body. The flexible shaft 22 may include a working channel through which elongated structures can be moved and, thus, positioned at the treatment zone within the cavity of the body.

The first generator 16 and the second generator 18 are each configured to generate electromagnetic energy of a fixed frequency. However, the generator unit 14 is not limited thereto; the first generator 16 and/or the second generator 18 can be configured to generate AC electromagnetic energy in a continuous range between a minimum frequency and a maximum frequency. The frequency of the electromagnetic energy to be generated by the first generator 16 and/or the second generator 18 may be selected using an interface (not shown in the figures).

The generator unit 14 can include a combiner 26 which is configured to temporally switch between outputting the output of the first generator 16 or the output of the second generator 18. The combiner 26 may also be configured to combine the outputs of the first generator 16 and of the second generator 18. In this case, the combiner 26 acts as a multiplexer or diplexer.

The generator unit 14 is thus capable of generating and controlling power to be delivered to the electrosurgical instrument 12, e.g. via a transmission line 28, which extends from the generator unit 14 through the surgical scoping device 19 and instrument channel to the distal tip of the instrument channel. The generator unit 14 may have a user interface for selecting and/or controlling the power delivered to the electrosurgical instrument 12, e.g. controlling the first and/or the second generators 16, 18 and/or the combiner 26. The generator unit 14 may have a display for showing the selected energy delivery mode. In some examples, the generator unit 14 may allow for an energy delivery mode to be selected based on the size of the vessel to be sealed.

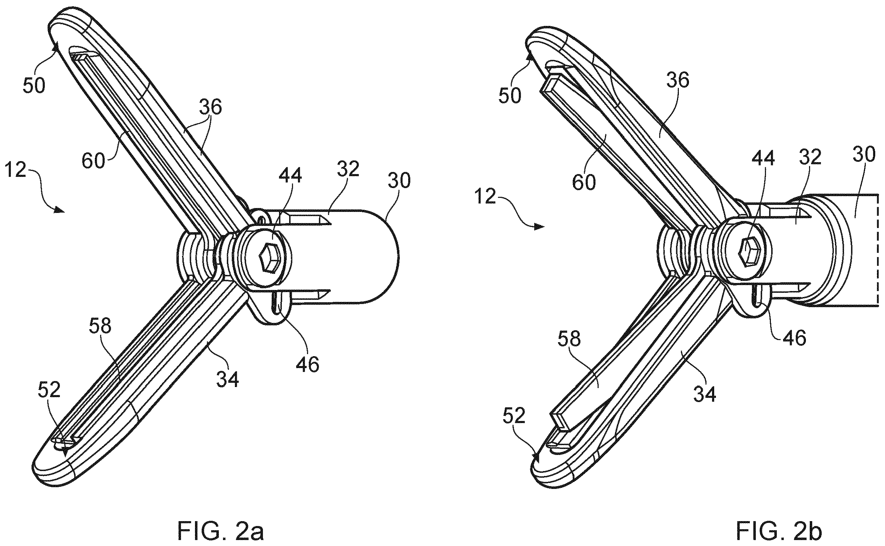

As shown in Figs. 1 and 2, the electrosurgical instrument 12 can include the transmission line 28, an instrument shaft (not shown in the figures), a joint (not shown in the figures), a first jaw 34, and/or a second jaw 36 which will be discussed in further detail below. The transmission line 28 may include a single coaxial cable that connects the generator unit 14 to the first jaw 34 and/or second jaw 34 for conveying the radiofrequency and/or microwave energy.

The first jaw 34 and the second jaw 36 are operably coupled to the joint 32 that is mounted on a distal end of the instrument shaft. Both the first and second jaws 34, 36 may be arranged to pivot relative to the joint. The joint may be arranged to ensure that the jaws remain laterally aligned as they are moved together.

In an alternative embodiment, the pair of jaws 34, 36 comprises a static jaw that is fixed relative to the instrument shaft or the joint. The other jaw is pivotable or rotatable.

The joint includes a pivot axle 44 which defines a pivot axis. The first jaw 34 and the second jaw 36 can pivot around the pivot axis or pivot axle 44. For example, the pivot axle 44 is fixed to the joint and the first jaw 34 and the second jaw 36 can rotate around the pivot axle 44.

The joint may have a clevis structure and slots on the arms of the clevis structure. The slot may have an elongate shape and can be a through-hole or a recess in an inner surface of the clevis structure. The first jaw 34 and/or the second jaw 36 also include elongated slots 46. A control wire or actuation rod includes a cam which is inserted in the slots of the clevis structure and in the slots 46 of the first jaw 34 and the second jaws 36. The engagement of the cam with the slots provides an actuation mechanism which translates a back-and-forth movement of the control wire (and thus of the cam) into a rotational movement of the first jaw 34 and the second jaw 36 around the pivot axle 44.

In use, the first jaw 34 and the second jaw 36 are intended to grip biological tissue (in particular a blood vessel) therebetween. The first jaw 34 and the second jaw 36 are arranged to apply pressure to the biological tissue between the opposed surfaces of the jaws 34, 36 and deliver energy (preferably microwave and/or radiofrequency electromagnetic energy) into the tissue from the transmission line 28.

The first jaw 34 includes a first surface 50 which opposes a second surface 52 of the second jaw 36. The first surface 50 and/or the second surface 52 may form an outer surface of the first jaw 34 and the second jaw 36 respectively, which can be brought into contact with each other when the jaws 34, 36 are in the closed position (see Fig. 5). For example, the first surface 50 and the second surface 52 may be considered pressure pads or pressure areas with which pressure can be applied to the tissue grasped between the first jaw 34 and the second jaw 36.

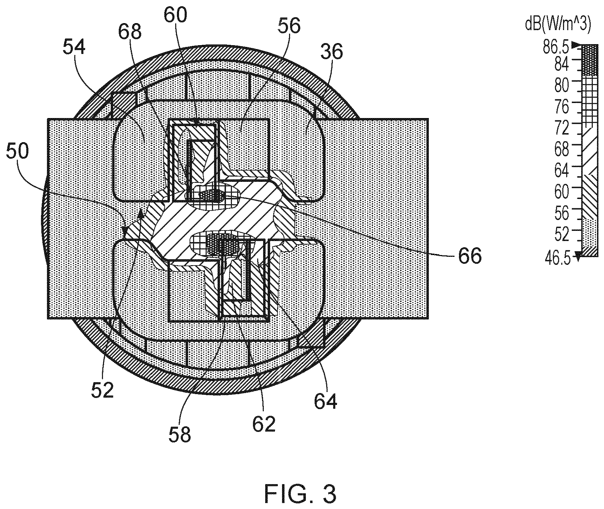

As best seen in Fig. 3, the first jaw 34 and the second jaw 36 include a shell 54 and a filler portion 56. The shell 54 and/or the filler portion 56 can be made from an electrically conductive material, such as metal or metal alloy. The shell 54 may provide a cavity within which the filler portion 56 is arranged. The shell 54 and the filler portion 56 of the first jaw 34 define a first recessed portion in which a first blade element 58 is arranged in the rest position. The shell 54 and the filler portion 56 of the second jaw 36 define a second recessed portion in which a second blade element 60 is arranged in the rest position. The filler portion 56 of the first jaw 34 is arranged in a position that is opposite the second blade element 60 in the rest position. The filler portion 56 of the second jaw 36 is arranged in a position that is opposite the first blade element 58 in the rest position. As such, the first jaw 34 and of the second jaw 36 may be point symmetric in the closed position.

Surface sections of the shell 54 and the filler portion 56 of the first jaw 34 that face the second jaw 36 or the second surface 52 may form the first surface 50. Surface sections of the shell 54 and the filler portion 56 of the second jaw 36 that face the first jaw 34 or the first surface 50 may form the second surface 52. The first surface 50 and the second surface 52 may be used for clamping or grasping tissue in the closed position in which the first surface 50 and the second surface 52 are in contact with each other.

The first blade element 58 and/or the second blade element 60 each include a dielectric planar body 62, an arm support 64, a first electrode 66, and/or a second electrode 68.

The first blade element 58 and the second blade element 60 may define a scissor-type closure mechanism in which tissue located in a gap between the blade elements 58, 60 when in the open position can have pressure applied to it as first blade element 58 and second blade element 60 are moved to a cutting position.

The planar dielectric body 62 may be made from ceramic or other suitable electrically insulating material. The planar dielectric body 62 defines a plane that is parallel to a plane through which the second blade element 60 pivots. The planar dielectric body 62 provides an insulating barrier between the first electrode 66 and the second electrode 68. For example, the second blade element 60 is arranged to slide past a first surface of the planar dielectric body 62, and the first electrode 66 is formed on this surface of the planar dielectric body 62 (this surface is an example of a first laterally facing surface). The first electrode 66 may be made from a conductor that exhibits high conductivity, e.g. gold or the like.

An exposed portion of the planar dielectric body 62 protrudes past the filler portion 56 in the rest position of the respective blade elements 58, 60. The first electrode 66 is arranged on that exposed portion of the planar dielectric body 62 or the first laterally facing surface that is exposed or that does not face the filler portion 56 in the rest position. As such, the first electrode 66 is completely exposed in the rest position.

The second electrode 68 extends along a second side surface of the planar dielectric body 62, the second surface being on the opposite side of the planar dielectric body 62 from the first side surface; this surface is an example of a second laterally facing surface. The second electrode 68 may be made from a conductor that exhibits high conductivity, e.g. gold or the like. The second laterally facing surface of the planar dielectric body 62 faces the arm support 64. So, the second electrode 68 is sandwiched between the arm support 64 and the planar dielectric body 62. However, the second electrode is exposed between the arm support 64 and the planar dielectric body 62 to be configured to contact tissue.

The first blade element 58 and the second blade element 60 may be positioned point-symmetric in the closed position of the jaws 34, 36 and the rest position of the blade elements 58, 60.

A section of the planar dielectric body 62 that is exposed between the first electrode 66 and the second electrode 68 may be flush with a section of the arm support 64 that faces the filler portion 56 on the respective other jaw. These sections of the planar dielectric body 62 and the arm support may be planar and flush with the first surface 50 or the second surface 52.

The first electrode 66 is electrically connected to an inner conductor of the coaxial transmission line 28 and the second electrode 68 is electrically connected to an outer conductor of the coaxial transmission line 28. The electrosurgical instrument 12 thus provides an energy delivery structure that is operable to deliver RF energy along a current path (e.g. through tissue) between the first electrode and second electrode, or microwave energy through a microwave field emitted by the first electrode and second electrode.

The arm support 64 may be rotatably coupled to the pivot axle 44 and is freely movable. Thus, the first blade element 58 and the second blade element 60 can be moved independently from the first jaw 34 and the second jaw 36, as shown in Figs. 2a and 2b. In particular, the first blade element 58 and the second blade element 60 can protrude from the first surface 50 and the second surface 52, respectively, in the cutting position.

The electrosurgical instrument 12 may provide two operational modalities. In a first modality, the blade elements 58, 60 may be used to perform a grasping cut, i.e. a cut through tissue captured between the jaws 34, 36. In this modality cutting is done by a combination of physical pressure applied by closing the blade elements 58, 60 and radiofrequency electromagnetic energy applied during the closing process while the first jaw 34 and the second jaw 36 are held in the closed position. As such, the pressure for holding the tissue and the pressure for cutting the tissue can be different since the first blade element 58 and the second blade element 60 can be actuated independently from the actuation of the first jaw 34 and the second jaw 36.

In a second modality, the blade elements 58, 60 may be used to grasp and seal tissue, such as a blood vessel or the like. In this modality, microwave electromagnetic energy is delivered to the electrodes 58, 60, which set up a microwave field that acts to coagulate the tissue held within the blade elements. During sealing, the tissue can be held by the first surface 50 and the second surface 52. As visible in Fig. 3, the intensity of emitted microwave energy is highest at the first electrode 66.

## Claims

1. An electrosurgical instrument for sealing and cutting tissue, comprising
an instrument shaft comprising a transmission line (28) for conveying microwave and radio frequency electromagnetic energy;
a first jaw (34) attached to the instrument shaft and including a first surface (50),
a second jaw (36) attached to the instrument shaft and including a second surface (52),
a first blade element (58), and
a second blade element (60),
wherein the first jaw (34) and the second jaw (36) can be moved between an open position, in which the tissue can be inserted between the first surface (50) and the second surface (52), and a closed position, in which the first and second surfaces (50, 52) are brought together to clamp the tissue therebetween,
wherein the first jaw (34) includes a first recessed portion in which the first blade element (58) can be received in a rest position,
wherein the second jaw (36) includes a second recessed portion in which the second blade element (60)can be received in the rest position,
wherein the first blade element and/or the second blade comprises a longitudinally extending planar dielectric body having a first electrode on a first laterally facing surface thereof; and
a second electrode spaced away from the first electrode and electrically isolated therefrom by at least the planar dielectric body;
**characterized in that** the first blade element (58) and the second blade element (60) lie alongside each other in a cutting position,
wherein, in the cutting position, the second blade element (60) lies adjacent to the first laterally facing surface of the first blade element (58), and
wherein the first electrode (66) and the second electrode (68) are operable:
as active and return electrodes for delivering radiofrequency electromagnetic energy and
as microwave electrodes for emitting microwave electromagnetic energy.

2. The electrosurgical instrument of claim 1, further comprising a joint having a pivot axis, wherein the first jaw (34), the second jaw (36), the first blade element (58) and/or the second blade element (60) are rotatable around the pivot axis.

3. The electrosurgical instrument of claim 2, wherein the first blade element (58) is movable relative to the first jaw (34) and/or the second blade element (60) is movable relative to the second jaw (36).

4. The electrosurgical instrument of any preceding claim, wherein, in the rest position, the first blade element (58) is flush the first surface (50) and/or the second blade element (60) is flush with the second surface (52).

5. The electrosurgical instrument of any preceding claim, wherein the first jaw (34) is point-symmetrical to the second jaw (36).

6. The electrosurgical instrument of any preceding claim, wherein the first jaw (34) and/or the second jaw (36) includes a shell (54) and a filler portion (56), wherein the filler portion (56) is arranged in a cavity defined by the shell (54), and wherein the shell (54) and the filler portion (56) define the first recessed portion and/or the second recessed portion, respectively.

7. The electrosurgical instrument of any preceding claim, wherein, in the rest position, the first electrode (66) is completely exposed and configured to contact tissue.

8. The electrosurgical instrument of claim 7, wherein, in the rest position, a portion of the planar dielectric body (62) protrudes from the filler portion (56) and the first electrode (66) is arranged on the protruding portion of the planar dielectric body (62).

9. The electrosurgical instrument of any preceding claim, wherein the second electrode (68) is located on the second laterally facing surface of the planar dielectric body (62) of the first blade element (58) and/or the second blade element (60).

10. The electrosurgical instrument of any preceding claim, wherein the first blade element (58) is point-symmetrical to the second blade element (60) in the rest position and the closed position.

11. The electrosurgical instrument of any preceding claim, wherein the first blade element (58) and/or the second blade element (60) each include a support arm to which the planar dielectric body (62) is attached, the support arm forming a part of an electrical connection between the transmission line (28) and the second electrode (68).

12. The electrosurgical instrument of claim 11 when dependent on any one of the claims 2 to 10, wherein the support arm is attached to the joint rotatably around the pivot axis.

13. The electrosurgical instrument of claims 11 or 12, wherein the second electrode (68) is arranged between the planar dielectric body (62) and the support arm and is exposed between the planar dielectric body (62) and the support arm so that the second electrode (68) is configured to contact tissue.

14. An electrosurgical apparatus for sealing and cutting tissue, comprising
a generator unit (14) for generating radiofrequency and/or microwave electromagnetic energy, **characterized by**
the electrosurgical instrument (12) according to any preceding claim,
wherein the transmission line (28) for conveys the radiofrequency and/or microwave electromagnetic energy from the generator unit (14) to the first electrode (66) and/or the second electrode (68).

15. The electrosurgical apparatus of claim 14, wherein the generator unit (14) is further configured to simultaneously or alternatingly generate microwave electromagnetic energy of the first frequency and radiofrequency electromagnetic energy of a second frequency.

## Patentansprüche

1. Elektrochirurgisches Instrument zum Versiegeln und Schneiden von Gewebe, umfassend
einen Instrumentenschaft, umfassend eine Übertragungsleitung (28) zum Übertragen von elektromagnetischer Mikrowellen- und Hochfrequenzenergie;
eine erste Backe (34), die an dem Instrumentenschaft befestigt ist und eine erste Oberfläche (50) umfasst,
eine zweite Backe (36), die an dem Instrumentenschaft befestigt ist und eine zweite Oberfläche (52) umfasst,
ein erstes Klingenelement (58) und
ein zweites Klingenelement (60),
wobei die erste Backe (34) und die zweite Backe (36) zwischen einer offenen Stellung, in welcher das Gewebe zwischen der ersten Oberfläche (50) und der zweiten Oberfläche (52) eingebracht sein können, und einer geschlossenen Stellung, in welcher die erste und die zweite Oberfläche (50, 52) zusammengebracht sind, um das Gewebe dazwischen einzuklemmen, bewegt werden können,
wobei die erste Backe (34) einen ersten ausgesparten Abschnitt umfasst, in dem das erste Klingenelement (58) in einer Ruhestellung aufgenommen sein kann,
wobei die zweite Backe (36) einen zweiten ausgesparten Abschnitt umfasst, in dem das zweite Klingenelement (60) in der Ruhestellung aufgenommen sein kann,
wobei das erste Klingenelement und/oder das zweite Klingenelement einen sich der Länge nach erstreckenden, planaren dielektrischen Körper umfasst/umfassen, der auf einer ersten, seitwärts gerichteten Oberfläche desselben eine erste Elektrode aufweist; und
wobei eine zweite durch zumindest den planaren dielektrischen Körper von der ersten Elektrode beabstandet und von dieser elektrisch isoliert ist;
**dadurch gekennzeichnet, dass** das erste Klingenelement (58) und das zweite Klingenelement (60) in einer Schneidstellung nebeneinander liegen,
wobei in der Schneidstellung das zweite Klingenelement (60) benachbart zu der ersten seitwärts gerichteten Oberfläche des ersten Klingenelements (58) liegt, und
wobei die erste Elektrode (66) und die zweite Elektrode (68) folgendermaßen betreibbar sind:
als eine aktive und eine Gegenelektrode zum Zuführen von elektromagnetischer Hochfrequenzenergie und
als Mikrowellenelektroden zum Emittieren von elektromagnetischer Mikrowellenenergie.

2. Elektrochirurgisches Instrument nach Anspruch 1, ferner umfassend ein Gelenk mit einer Schwenkachse, wobei die erste Backe (34), die zweite Backe (36), das erste Klingenelement (58) und/oder das zweite Klingenelement (60) um die Schwenkachse herum drehbar sind.

3. Elektrochirurgisches Instrument nach Anspruch 2, wobei das erste Klingenelement (58) relativ zu der ersten Backe (34) bewegbar ist und/oder das zweite Klingenelement (60) relativ zu der zweiten Backe (36) bewegbar ist.

4. Elektrochirurgisches Instrument nach einem der vorangegangenen Ansprüche, wobei in der Ruhestellung das erste Klingenelement (58) bündig mit der ersten Oberfläche (50) ist und/oder das zweite Klingenelement (60) bündig mit der zweiten Oberfläche (52) ist.

5. Elektrochirurgisches Instrument nach einem der vorangegangenen Ansprüche, wobei die erste Backe (34) punktsymmetrisch zu der zweiten Backe (36) ist.

6. Elektrochirurgisches Instrument nach einem der vorangegangenen Ansprüche, wobei die erste Backe (34) und/oder die zweite Backe (36) eine Hülle (54) und einen Füllabschnitt (56) umfasst, wobei der Füllabschnitt (56) in einem durch die Hülle (54) definierten Hohlraum angeordnet ist, und wobei die Hülle (54) und der Füllabschnitt (56) jeweils den ersten ausgesparten Abschnitt bzw. den zweiten ausgesparten Abschnitt definieren.

7. Elektrochirurgisches Instrument nach einem der vorangegangenen Ansprüche, wobei in der Ruhestellung die erste Elektrode (66) vollständig freigelegt und zum Inkontaktbringen mit Gewebe ausgelegt ist.

8. Elektrochirurgisches Instrument nach Anspruch 7, wobei in der Ruhestellung ein Abschnitt des planaren dielektrischen Körpers (62) von dem Füllabschnitt (56) wegsteht und die erste Elektrode (66) auf dem wegstehenden Abschnitt des planaren dielektrischen Körpers (62) angeordnet ist.

9. Elektrochirurgisches Instrument nach einem der vorangegangenen Ansprüche, wobei die zweite Elektrode (68) auf der zweiten seitwärts gerichteten Oberfläche des planaren dielektrischen Körpers (62) des ersten Klingenelements (58) und/oder des zweiten Klingenelements (60) angeordnet ist.

10. Elektrochirurgisches Instrument nach einem der vorangegangenen Ansprüche, wobei das erste Klingenelement (58) in der Ruhestellung und in der geschlossenen Stellung punktsymmetrisch zu dem zweiten Klingenelement (60) ist.

11. Elektrochirurgisches Instrument nach einem der vorangegangenen Ansprüche, wobei das erste Klingenelement (58) und/oder das zweite Klingenelement (60) jeweils einen Stützarm umfassen, an dem der planare dielektrische Körper (62) befestigt ist, wobei der Stützarm einen Teil einer elektrischen Verbindung zwischen der Übertragungsleitung (28) und der zweiten Elektrode (68) ausbildet.

12. Elektrochirurgisches Instrument nach Anspruch 11 in Abhängigkeit von einem der Ansprüche 2 bis 10, wobei der Stützarm an dem Gelenk um die Schwenkachse drehbar befestigt ist.

13. Elektrochirurgisches Instrument nach Anspruch 11 oder 12, wobei die zweite Elektrode (68) zwischen dem planaren dielektrischen Körper (62) und dem Stützarm angeordnet ist und zwischen dem planaren dielektrischen Körper (62) und dem Stützarm derart freigelegt ist, dass die zweite Elektrode (68) zum Inkontaktbringen mit Gewebe ausgelegt ist.

14. Elektrochirurgische Vorrichtung zum Versiegeln und Schneiden von Gewebe, umfassend
eine Generatoreinheit (14) zum Erzeugen von elektromagnetischer Hochfrequenz- und/oder Mikrowellenenergie, **gekennzeichnet durch**
ein elektrochirurgisches Instrument (12) nach einem der vorangegangenen Ansprüche,
wobei die Übertragungsleitung (28) die elektromagnetische Hochfrequenz- und/oder Mikrowellenenergie von der Generatoreinheit (14) zu der ersten Elektrode (66) und/oder der zweiten Elektrode (68) befördert.

15. Elektrochirurgische Vorrichtung nach Anspruch 14, wobei die Generatoreinheit (14) ferner dazu ausgelegt ist, gleichzeitig oder abwechselnd elektromagnetische Mikrowellenenergie der ersten Frequenz und elektromagnetische Hochfrequenzenergie einer zweiten Frequenz zu erzeugen.

## Revendications

1. Instrument électrochirurgical pour sceller et découper un tissu, comprenant
une tige d'instrument comprenant une ligne de transmission (28) pour transporter une énergie électromagnétique à micro-ondes et radiofréquence ;
une première mâchoire (34) fixée à la tige d'instrument et incluant une première surface (50),
une seconde mâchoire (36) fixée à la tige d'instrument et incluant une seconde surface (52),
un premier élément de lame (58), et
un second élément de lame (60),
dans lequel la première mâchoire (34) et la seconde mâchoire (36) peuvent être déplacées entre une position ouverte, dans laquelle le tissu peut être inséré entre la première surface (50) et la seconde surface (52), et une position fermée, dans laquelle les première et seconde surfaces (50, 52) sont rapprochées pour serrer le tissu entre celles-ci,
dans lequel la première mâchoire (34) inclut une première partie évidée dans laquelle le premier élément de lame (58) peut être reçu dans une position de repos,
dans lequel la seconde mâchoire (36) inclut une seconde partie évidée dans laquelle le second élément de lame (60) peut être reçu dans la position de repos.
dans lequel le premier élément de lame et/ou le second élément lame comprennent un corps diélectrique plan s'étendant longitudinalement présentant une première électrode sur une première surface orientée latéralement de celui-ci ; et
une seconde électrode espacée de la première électrode et isolée électriquement de celle-ci par au moins le corps diélectrique plan ;
**caractérisé en ce que** le premier élément de lame (58) et le second élément de lame (60) se trouvent l'un à côté de l'autre dans une position de coupe,
dans lequel, dans la position de coupe, le second élément de lame (60) est adjacent à la première surface orientée latéralement du premier élément de lame (58), et
dans lequel la première électrode (66) et la seconde électrode (68) peuvent fonctionner :
en tant qu'électrodes actives et de retour pour délivrer de l'énergie électromagnétique radiofréquence et
en tant qu'électrodes à micro-ondes pour émettre une énergie électromagnétique à micro-ondes.

2. Instrument électrochirurgical selon la revendication 1, comprenant en outre une articulation présentant un axe de pivotement, dans lequel la première mâchoire (34), la seconde mâchoire (36), le premier élément de lame (58) et/ou le second élément de lame (60) peuvent tourner autour de l'axe de pivotement.

3. Instrument électrochirurgical selon la revendication 2, dans lequel le premier élément de lame (58) est mobile par rapport à la première mâchoire (34) et/ou le second élément de lame (60) est mobile par rapport à la seconde mâchoire (36).

4. Instrument électrochirurgical selon l'une quelconque des revendications précédentes, dans lequel, dans la position de repos, le premier élément de lame (58) affleure la première surface (50) et/ou le second élément de lame (60) affleure la seconde surface (52).

5. Instrument électrochirurgical selon l'une quelconque des revendications précédentes, dans lequel la première mâchoire (34) est à symétrie ponctuelle par rapport à la seconde mâchoire (36).

6. Instrument électrochirurgical selon l'une quelconque des revendications précédentes, dans lequel la première mâchoire (34) et/ou la seconde mâchoire (36) inclut une coque (54) et une partie de remplissage (56), dans lequel la partie de remplissage (56) est agencée dans une cavité définie par la coque (54), et dans lequel la coque (54) et la partie de remplissage (56) définissent la première partie évidée et/ou la seconde partie évidée, respectivement.

7. Instrument électrochirurgical selon l'une quelconque des revendications précédentes, dans lequel, dans la position de repos, la première électrode (66) est complètement exposée et configurée pour venir en contact avec du tissu.

8. Instrument électrochirurgical selon la revendication 7, dans lequel, dans la position de repos, une partie du corps diélectrique plan (62) fait saillie à partir de la partie de remplissage (56) et la première électrode (66) est agencée sur la partie en saillie du corps diélectrique plan (62).

9. Instrument électrochirurgical selon l'une quelconque des revendications précédentes, dans lequel la seconde électrode (68) se trouve sur la seconde surface orientée latéralement du corps diélectrique plan (62) du premier élément de lame (58) et/ou du second élément de lame (60).

10. Instrument électrochirurgical selon l'une quelconque des revendications précédentes, dans lequel le premier élément de lame (58) est à symétrie ponctuelle par rapport au second élément de lame (60) dans la position de repos et la position fermée.

11. Instrument électrochirurgical selon l'une quelconque des revendications précédentes, dans lequel le premier élément de lame (58) et/ou le second élément de lame (60) comprennent chacun un bras de support auquel le corps diélectrique plan (62) est fixé, le bras de support formant une partie d'une connexion électrique entre la ligne de transmission (28) et la seconde électrode (68).

12. Instrument électrochirurgical selon la revendication 11 lorsqu'elle dépend de l'une quelconque des revendications 2 à 10, dans lequel le bras de support est fixé à l'articulation de manière rotative autour de l'axe de pivotement.

13. Instrument électrochirurgical selon la revendication 11 ou 12, dans lequel la seconde électrode (68) est agencée entre le corps diélectrique plan (62) et le bras de support et est exposée entre le corps diélectrique plan (62) et le bras de support de telle sorte que la seconde électrode (68) soit configurée pour venir en contact avec du tissu.

14. Appareil électrochirurgical pour sceller et découper du tissu, comprenant
une unité de génération (14) pour générer de l'énergie électromagnétique radiofréquence et/ou à micro-ondes, **caractérisé par**
l'instrument électrochirurgical (12) selon l'une quelconque des revendications précédentes,
dans lequel la ligne de transmission (28) transporte l'énergie électromagnétique radiofréquence et/ou à micro-ondes à partir de l'unité de génération (14) vers la première électrode (66) et/ou la seconde électrode (68).

15. Appareil électrochirurgical selon la revendication 14, dans lequel l'unité de génération (14) est en outre configurée pour générer simultanément ou alternativement une énergie électromagnétique à micro-ondes de la première fréquence et une énergie électromagnétique radiofréquence d'une seconde fréquence.
